(19) Europäisches Patentamt
European Patent Office
Office européen des brevets

(11) Publication number: **0 285 364 B1**

(12) **EUROPEAN PATENT SPECIFICATION**

(45) Date of publication of patent specification: **15.12.93**

(51) Int. Cl.[5]: **A61K 7/06**

(21) Application number: **88302767.4**

(22) Date of filing: **29.03.88**

(54) **Use of a hair cosmetic composition for preventing generation of split hair or torn hair and for repairing damaged hair.**

(30) Priority: **30.03.87 JP 77048/87**
**01.07.87 JP 164936/87**
**30.03.87 JP 77047/87**

(43) Date of publication of application:
**05.10.88 Bulletin 88/40**

(45) Publication of the grant of the patent:
**15.12.93 Bulletin 93/50**

(84) Designated Contracting States:
**DE FR GB IT NL**

(56) References cited:
**EP-A- 0 155 806**

(73) Proprietor: **SHISEIDO COMPANY LIMITED**
**5-5 Ginza 7-chome**
**Chuo-ku Tokyo(JP)**

(72) Inventor: **Nanba, Tomiyuki**
**c/o Shiseido Company Ltd.,**
**7-5-5, Ginza**
**Chuo-ku, Tokyo(JP)**
Inventor: **Torii, Kenji**
**c/o Shiseido Company Ltd.,**
**7-5-5, Ginza**
**Chuo-ku, Tokyo(JP)**
Inventor: **Yasuhara, Hiroaki**
**c/o Shiseido Company Ltd.,**
**7-5-5, Ginza**
**Chuo-ku, Tokyo(JP)**
Inventor: **Tomita, Kenichi**
**c/o Shiseido Company Ltd.,**
**7-5-5, Ginza**
**Chuo-ku, Tokyo(JP)**
Inventor: **Fukuchi, Yoko**
**c/o Shiseido Company Ltd.,**
**7-5-5, Ginza**
**Chuo-ku, Tokyo(JP)**

(74) Representative: **Hale, Stephen Geoffrey et al**
**J.Y. & G.W. Johnson**
**Furnival House**
**14/18 High Holborn**
**London WC1V 6DE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

BACKGROUND OF THE INVENTION

1. Field of the Invention

The present invention relates to the use of a hair cosmetic composition giving an excellent luster to the hair, having a good set retentive force, imparting a smooth feeling to the hair, for preventing the generation of split hair and torn hair, and for repairing hair.

Generally speaking, when complicated beauty treatments involving hair washing, brushing, heat by a dryer, hair coloring, bleaching agents, etc. are applied repeatedly to the hair, the hair will be extensively damaged and deteriorated and, consequently, becomes dry and unkempt, or an increased splitting of hair or of torn hair and a lowering in strength occur, as is well known in the art.

Accordingly, to alleviate such hair damage as described above, and to protect and repair hair, oil components such as silicone oil, ester oil, hydrocarbon oil, etc. have been used as solubilized, emulsified or dissolved as the base in hair cosmetics to give a luster and smoothness to the hair. Particularly, silicone oil having a degree of polymerization of 3 to 650 has a low surface tension and an excellent compatibility with hair to give a good luster thereto, and therefore, has been frequently used recently. However, the oil component is limited, and if used in a large amount or used over a long period, a drawback arises in that the hair becomes fatty. Also, cationic surfactants have been frequently used for imparting smoothness, but a desirable luster cannot be given thereby, and a drawback arises in an absence of undesirable side effects cannot be guaranteed when these surfactants are formulated in a large amount. EP-A-155806 discloses a hair conditioning composition containing as essential ingredients (a) a silicone conditioning agent, preferably a linear polydimethylsiloxane of the formula:

$$(CH_2)_3\ SiO\{Si(CH_3)_2O\}_n\ Si(CH_3)_3$$

where n is from 1 to 15000, preferably from 20 to 7000, and unsubstituted or substituted by vinyl, phenyl, carboxyl, alkoxy, mercapto, alkyl or amino, (b) a dimethicone copolyol, (c) a lipid vehicle material, (d) a cationic surfactant vehicle material and (e) water. These compositions are said to have improved hair-care benefits while maintaining excellent in-use characteristics, in particular to exhibit lower resoiling of hair after use. No mention is made, however, of preventing generation of split or torn hair or of repairing damaged hair.

Also, for hair setting polymeric compounds, such as polyvinyl pyrrolidone type polymers, acidic polyvinyl ether type polymers, acidic acrylic polymers or cationic polymers, etc. have been employed. Although these gave an excellent fixing, namely hair setting power, they did not give a satisfactory luster and smoothness, and had a drawback of hardening the hair, i.e., making it stiff.

Starting materials extracted from natural products had some effects in hair damage prevention and protection from chemical or mechanical treatments, but none were satisfactory because, for example, the adhesiveness to the hair was weak or a feeling such as smoothness after use was unsatisfactory.

The present invention seeks to provide a means of preventing the generation of split hair or torn hair, and of repairing hair, preferably employing a hair cosmetic composition which gives an excellent luster, imparts a smooth feeling to the hair without fattiness and stiffness.

Other advantages of the present invention will be apparent from the following description.

In accordance with the present invention, there is provided the use for preventing the generation of split hair or torn hair and for repairing damaged hair of a hair cosmetic composition comprising at least one high molecular weight silicone having the formula (I):

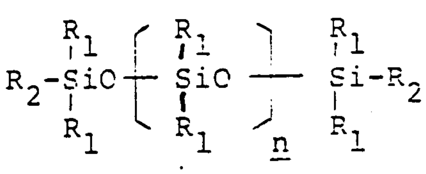

(I)

where each $R_1$ represents methyl or one or more of the $R_1$s represents phenyl and the remainder represent methyl, each $R_2$ represents methyl or hydroxy, and n represents an integer of 3000 to 20000, said composition being free of any dimethicone copolyol. The invention also includes the use of such a silicone

for the production of such a hair cosmetic composition.

For the use in accordance with the present invention, there are provided various preferred hair cosmetic compositions containing, as an essential component, the above-mentioned high molecular weight silicone (I) and other components as follows:

(1)

(A) 0.1% to 25% by weight of the above-mentioned high molecular weight silicone (I);
(B) 1% to 98.9% by weight of at least one cyclic silicone; and
(C) 1% to 98.9% by weight of trichlorotrifluoroethane and/or tetrachlorodifluoroethane;

(2)

(A) 0.1% to 25% by weight of the above-mentioned high molecular weight silicone (I):
(B) 45% to 98.9% by weight of at least one cyclic silicone; and
(C) 1% to 30% by weight of at least one lower alcohol;

(3)

(A) 0.01% to 25% by weight of the above-mentioned high molecular weight silicone (I):
(B) 1% to 98.9% by weight of trichlorotrifluoroethane and/or tetrachlorodifluoroethane; and
(C) 5% to 95% by weight of an aerosol propellant.

The high molecular weight silicone (I) usable for the present invention has a high molecular weight and the value of n in the formula (I) is 3000 to 20000. The molecular weight is about 370000 to 1500000, and soft rubbery properties are exhibited at normal temperature.

Typical examples of such silicones are dimethylpolysiloxane, methylphenylpolysiloxane, dimethylpolysiloxane containing terminal hydroxyl groups, and methylphenylpolysiloxane containing terminal hydroxyl groups.

The use of the high molecular weight silicone according to the invention prevents and repairs damage such as split hair and torn hair by coating the damaged portion of the hair surface with a thin film of a high molecular weight silicone.

The amount of the high molecular weight silicone formulated may be 0.1% to 50% by weight, preferably 1% to 30% by weight, in the total amount of the cosmetic composition. When the amount is less than 0.1% by weight, a satisfactory effect will not be obtained. When the amount exceeds 50% by weight, solubility is achieved only with difficulty.

The high molecular weight silicone is formulated in a hair cosmetic composition, preferably by dissolving in a volatile or a low boiling-point oil, or it may be formulated separately into the hair cosmetic compositions and dissolved in the system.

Examples of the low boiling-point oil include low boiling-point linear silicones, low boiling-point cyclic silicones and low boiling-point isoparaffinic hydrocarbons.

The low boiling-point linear silicone is represented by the formula:

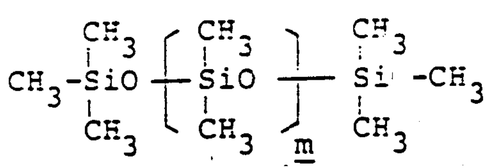

where m represents 0 or an integer of 1 to 5. Specific examples thereof include hexamethyldisiloxane, octamethyltrisiloxane, decamethyltetrasiloxane and hexadecamethylheptasiloxane.

The low boiling-point cyclic silicone is represented by the formula:

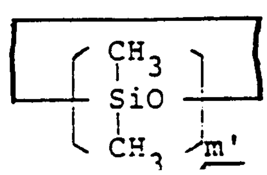

where m' represents an integer of 3 to 7. Specific examples thereof include octamethylcyclotetrasiloxane, decamethylcyclopentasiloxane and tetradecamethylcyclohexasiloxane.

The low boiling-point isoparaffinic hydrocarbon may be an isoparaffinic hydrocarbon with boiling points at normal pressure in the range of from 60 °C to 260 °C, as exemplified by Isopar (trademark) A, C, D, E, G, H, K. L, M produced by Exxon Co., Shellsol 71 (trademark) produced by Shell Co., Soltol (trademark) 100,

130 or 220 produced by Phillips Co.

The above-mentioned low boiling-point oil may be used alone or any mixture thereof and the total amount formulated is preferably 1 to 50-fold by weight relative to the high molecular weight silicone, preferably 10% to 80% by weight, in the total weight of the hair cosmetic composition.

Any desired agent form may be used in the present invention, and any of the solubilized system, the emulsified system, the powder dispersion system, the two layer oil-water system, and the three layer oil-water-powder system may be used.

In the emulsified system, generally the oil phase containing the high molecular weight silicone is emulsified with a nonionic surfactant, a cationic surfactant, an anionic surfactant or a mixture thereof. In this case, it is preferable to use the method in which a mixture of surfactants with a water-soluble polyhydric alcohol is previously prepared and then mixed with the oil phase, to obtain an emulsifier composition.

The water-soluble polyhydric alcohols usable in the present invention are polyhydric alcohols having two or more hydroxyl groups in the molecule. Typical examples of such polyhydric alcohols are dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; pentahydric alcohols such as xylytol, etc.; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentanerythritol ether. These polyhydric alcohols, which may be used alone or in any mixture thereof, may be selected and used as desired.

As the oil constituting the oil phase of the emulsified cosmetic, other than the high molecular weight silicone the following oils may be used,

namely, oil components generally employed in cosmetics compositions including liquid oils and fats such as avocado oil, tsubaki oil, turtle oil, Macademia nuts oil, corn oil, mink oil, olive oil, rape seed oil, yolk oil, sesame oil, parsic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, hohoba oil, germ oil, triglycerine, trioctanoic acid glycerine, triisopalmitic acid glycerine; solid fats such as cacao fat, coconut oil, horse fat, hardened coconut fat, palm oil, tallow, sheep fat, hardened tallow, palm kernel oil, lard, ox bone fat, wood wax kernel oil, hardened castor oil; waxes such as beeswax, canderilla wax, cotton wax, carunauba wax, bayberry wax, insect wax, whale wax, montan wax, rice bran wax, lanolin, kapok wax, lanolin acetate, liquid lanolin, cane wax, isopropyl lanolin fatty acid, hexyllaurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether; hydrocarbons such as fluid paraffin, ozocerite, squalene, pristan, paraffin, ceresin, squalane, petrolatum, microcrystalline wax; fatty acid oils, alcohols, ester oils such as cetyl octanoate, isopropyl myristate; silicone oils such as dimethylpolysiloxane, methylphenylpolysiloxane; silicone resins and the like.

The preferable formulated amounts of the above components may be 0.5% to 10% by weight in the total amount of the hair cosmetic for the surfactant, and 10% to 80% by weight for the oil phase containing

the high molecular weight silicone, and in the method of preparing previously a mixture of the surfactant and water-soluble polyhydric alcohol and mixing the mixture with the oil phase to give an emulsified composition, the surfactant may be 1% to 20% by weight, the oil phase containing the high molecular weight silicone is 10% to 70% by weight, and the water-soluble polyhydric alcohol is preferably 5% to 30% by weight in the total amount of the hair cosmetic, and about 50% to 99% by weight based on the surfactant.

The emulsification form of the emulsified cosmetic can be the water-in-oil form or oil-in-water form, but is desirably prepared so that the water repellency effect is not lost.

According to the second embodiment a hair cosmetic composition for use in the present invention contains (A) 0.1% to 25% by weight, preferably 0.5% to 15% by weight, in view of the viscosity of the composition, of the above-mentioned high molecular weight silicone (I); (B) 1% to 98.9% by weight of the above-mentioned cyclic silicone; and (C) 1% to 98.9% by weight, preferably 1% to 50% by weight, of trichlorotrifluoroethane and/or tetrachlorodifluoroethane in the total composition, and, optionally, lower alcohols such as ethanol, isopropyl alcohol are provided.

From the viewpoint of application of the hair cosmetic compositions to the hair, the viscosity thereof is generally less than 30000 cps, preferably 20 to 5000 cps.

According to the third embodiment a hair cosmetic composition for use in the present invention contains (A) 0.1% to 25% by weight, preferably 0.5 to 15% by weight, in view of the viscosity of the composition, of the above-mentioned high molecular weight silicone (I); (B) 45% to 98.9% by weight of the above-mentioned cyclic silicone; and (C) 1% to 30% by weight, preferably 5% to 15% by weight, of the lower alcohol such as ethanol, isopropyl alcohol.

From the viewpoint of application of the hair cosmetic compositions to the hair, the viscosity thereof is generally less than 30000, preferably 20 to 5000 cps. The use of too large an amount of the lower alcohol will not allow the dissolution of the high molecular weight silicone.

According to the fourth embodiment as the aerosol type hair cosmetic composition for use in the invention contains (A) 0.01% to 25% by weight, preferably 0.5% to 5% by weight, in view of the applicability of the composition to the hair, of the high molecular weight silicone (I); (B) 1% to 98.9% by weight, preferably 1% to 50% by weight of trichlorotrifluoroethane and/or tetrachlorodifluoroethane; and (C) 5% to 95% by weight, preferably 10% to 80% by weight of an aerosol propellant such as trichlorofluoromethane, dichlorodifluoromethane, dichlorotetrafluoroethane, monochlorodifluoromethane, dimethylether, propane, isobutane and/or n-butane; and, optionally, (D) a lower alcohol such as ethanol or isopropyl alcohol.

In the hair cosmetic composition for use in the present invention, in addition to the above-mentioned essential constituents, further ingredients such as UV-ray absorbers, anti-oxidants, preservatives, pharmaceutically active substances such as vitamins, hormones, etc., and flavors may be also formulated, depending on the purpose, within quantitative and qualitative ranges that will not impair the present invention.

## Examples

Hair cosmetic compositions for use in the present invention will now be further illustrated by, but are by no means limited to, the following Examples and Comparative Examples, wherein all percentages are expressed on a weight basis unless otherwise noted.

### Example 1: Hair Oil

| | |
|---|---|
| (1) Decamethylcyclopentasiloxane | 70.0% |
| (2) Dimethylpolysiloxane 5 cps | 20.0 |
| (3) Dimethylpolysiloxane ($R_1$ and $R_2$ in the formula (I) are methyl group, n = 7000) | 10.0 |
| (4) Flavor | q.s. |
| (1) - (4) were dissolved and mixed while stirring at 70 - 80 °C, and a viscous liquid hair oil with a viscosity of 500 cps and a good transparency was obtained. | |

Example 2: Hair Blow

| | |
|---|---|
| (1) Decamethylcyclopentasiloxane | 10.0% |
| (2) Methylphenylpolysiloxane (10% of $R_1$ in the formula (I) is phenyl group with remainder being methyl group, $R_2$ is methyl group, n = 15000) | 1.0 |
| (3) 1,3-Butylene glycol | 2.0 |
| (4) Polyethylene (60) hardened castor oil ester | 2.0 |
| (5) Ethyl alcohol 95% | 15.0 |
| (6) Purified water | 70.0 |
| (7) Flavor | q.s. |
| (4) was dissolved in (3), and emulsified by an addition of (1), (2) and mixed with (5), (6), (7). The composition was filled in a dispenser vessel, and sprayed in the atomized state onto the hair. | |

Example 3: Foamy Hair Setting Agent

| | |
|---|---|
| (1) Dimethylpolysiloxane 1.5 cps | 3.0% |
| (2) Dimethylpolysiloxane 20.0cps | 2.0 |
| (3) Dimethylpolysiloxane ($R_1$ in the formula (I) is methyl group, $R_2$ is hydroxy group, n = 5000) | 5.0 |
| (4) Glycerine | 3.0 |
| (5) Polyethylene glycol (120) hardened castor oil ester | 3.0 |
| (6) Behenyltrimethylammonium chloride | 0.7 |
| (7) Flavor | q.s. |
| (8) Purified water | 83.3 |
| (4) and (5) were dissolved, and emulsified with the addition of a mixture of (1), (2), (3), and then the emulsified product thus obtained was added and mixed into a mixture of (6), (7), (8). | |

Example 4: Hair Oil

| | |
|---|---|
| (1) Decamethylcyclopentasiloxane | 80.0% |
| (2) Dimethylpolysiloxane ($R_1$ and $R_2$ in the formula (I) are methyl groups, n = 4000) | 8.0 |
| (3) Organic silicone resin comprising $(CH_3)_3SiO_{1/2}SiO_2/(CH_3)_2SiO$ = 2.4/1.6 = 1.0 (molar ratio) | 2.0 |
| (4) Ethanol | 10.0 |
| (5) Flavor | q.s. |
| (1) - (5) were dissolved while stirring at 70 - 80 °C to obtain a liquid hair oil with a viscosity of 700 cps. | |

Example 5: Hair Oil

| | |
|---|---|
| (1) Decamethylcyclopentasiloxane | 40.0% |
| (2) Dimethylpolysiloxane ($R_1$ and $R_2$ in the formula (I) are methyl group, n = 3000) | 10.0 |
| (3) Trichlorotrifluoroethane | 50.0 |
| (1) - (3) were dissolved and mixed while stirring, and a viscous liquid hair oil with a viscosity of 200 cps and a good transparency was obtained. | |

Example 6: Hair Oil

| | |
|---|---|
| (1) Octamethylcyclotetrasiloxane | 20.0% |
| (2) Dimethylpolysiloxane ($R_1$ in the formula (I) is methyl group, $R_2$ hydroxyl group, n = 10000) | 0.5 |
| (3) Trichlorotrifluoroethane | 79.5 |

A liquid hair oil with a viscosity of 8 cps was obtained in the same manner as in Example 6.

Example 7: Hair Oil

| | |
|---|---|
| (1) Dodecamethylcyclohexanesiloxane | 30.0% |
| (2) Dimethylpolysiloxane (Methyl: Phenyl in $R_1$ in the formula (I) = 1:1, $R_2$ methyl, n = 20000) | 25.0 |
| (3) Isopropyl alcohol | 5.0 |
| (4) Trichlorotrifluoroethane | 20.0 |
| (5) Tetrachlorodifluoroethane | 20.0 |

A transparent liquid hair oil with a viscosity of 2000 cps was obtained in the same manner as in Example 6.

Example 8: Hair Oil

| | |
|---|---|
| (1) Decamethylcyclopentanesiloxane | 20.0% |
| (2) Octamethylcyclotetrasiloxane | 20.0 |
| (3) Ethanol (95%) | 25.0 |
| (4) Tetrachlorodifluoroethane | 20.0 |
| (5) Dimethylpolysiloxane ($R_1$ and $R_2$ in the formula (I) are methyl group, n = 15,000) | 15.0 |

A transparent liquid hair oil with a viscosity of 1500 cps was obtained in the same manner as in Example 6.

Example 9: Hair Oil

| | |
|---|---|
| (1) Decamethylcyclopentasiloxane | 70.0% |
| (2) Ethanol | 20.0 |
| (3) Dimethylpolysiloxane ($R_1$ and $R_2$ in the formula (I) are methyl group, n = 3000) | 10.0 |
| (4) Flavor | q.s. |
| (1) - (4) were dissolved and mixed while stirring, and a viscous liquid hair oil with a viscosity of 500 cps and a good transparency was obtained. | |

Example 10: Hair Oil

| | |
|---|---|
| (1) Octamethylcyclotetrasiloxane | 69.9% |
| (2) Dimethylpolysiloxane ($R_1$ in the formula (I) is methyl group, $R_2$ hydroxyl group, n = 10000) | 0.1 |
| (3) Ethanol (99%) | 30.0 |

A liquid hair oil with a viscosity of 20 cps was obtained in the same manner as in Example 9.

Example 11: Hair Oil

| | |
|---|---|
| (1) Dodecamethylcyclohexasiloxane | 45.0% |
| (2) Dimethylpolycyclohexane (Methyl: Phenyl in $R_1$ in the formula (I) = 1:1, $R_2$ methyl group, n = 20000) | 25.0 |
| (3) Isopropylalcohol | 30.0 |
| (4) Flavor | q.s. |

A liquid hair oil with a viscosity of 5000 cps was obtained in the same manner as in Example 9.

Example 12: Hair Oil

| | |
|---|---|
| (1) Decamethylcyclopentasiloxane | 40.0% |
| (2) Octamethylcyclotetrasiloxane | 40.0 |
| (3) Dimethylpolysiloxane ($R_1$ and $R_2$ are methyl group, n = 5000) | 5.0 |
| (4) Dimethylpolysiloxane (Methyl: phenyl in $R_1$ of the formula (I) = 1:1, $R_2$ hydroxyl, n = 15000) | 10.0 |
| (5) Ethanol (95%) | 2.0 |
| (6) Isopropylalcohol | 3.0 |

A transparent liquid hair oil with a viscosity of 2500 cps was obtained in the same manner as in Example 9.

Example 13: Hair Spray

| | |
|---|---|
| (1) Dimethylpolysiloxane (Methyl: phenyl in the formula (I) = 1:1, $R_2$ methyl, n = 10000) | 0.5% |
| (2) Trichlorotrifluoroethane | 50.0 |
| (3) Trichlorofluoromethane | 20.0 |
| (4) Dichlorodifluoromethane | 30.0 |
| (1) and (2) were dissolved mixed while stirring and the resultant mixture was charged into an aersol | |

container. After attaching a valve, (3) and (4) were added therein.

Example 14: Hair Spray

| | |
|---|---|
| (1) Dimethylpolysiloxane ($R_1$ and $R_2$ in the formula (I) are methyl group, n = 3000) | 2.5% |
| (2) Dimethylpolysiloxane ($R_1$ is methyl, $R_2$ hydroxyl, n = 20,000) | 2.5 |
| (3) Tetrachlorodifluoroethane | 10.0 |
| (4) Trichlorotrifluoroethane | 5.0 |
| (5) Dodecamethylcyclohex siloxane | 5.0 |
| (6) Isoper | 10.0 |
| (7) Ethanol | 5.0 |
| (8) Flavor | q.s. |
| (9) Diethyl ether | 30 |
| (10) n-Butane | 30 |
| (1) - (8) were dissolved and mixed while stirring and the resultant mixture was charged into an aerosol container. After attaching a valve, (9) and (10) were added therein. | |

Example 15: Hair Spray

| | |
|---|---|
| (1) Dimethylpolysiloxane ($R_1$ and $R_2$ in the formula (I) are methyl, n = 10,000) | 0.5% |
| (2) Tetrachlorodifluoroethane | 2.0 |
| (3) Decamethylcyclopentasiloxane | 75.5 |
| (4) Ethanol | 2.0 |
| (5) Isopropylalcohol | 10.0 |
| (6) Flavor | q.s. |
| (7) Propane | 7.0 |
| (8) Dimethyl ether | 3.0 |
| (1) - (6) were dissolved and mixed while stirring and the resultant mixture was charged into an aerosol container. After attaching a valve, (1) and (8) were added therein. | |

The hair cosmetic compositions according to Examples 1 to 15 of the present invention gave an excellent luster and smoothness to the hair and hair damage preventing and repairing effects, and had an strong hair setting power.

Example 16: Hair Blow

| | |
|---|---|
| (1) Decamethylcyclopentasiloxane | 15% |
| (2) Dimethylpolysiloxane ($R_1$ and $R_2$ in the formula (I) are methyl groups, n = 3000) | 4 |
| (3) 1,3-butylene glycol | 2 |
| (4) Polyethylene (60) hardened castor oil ester | 2 |
| (5) Ethanol | 15 |
| (6) Deionized water | 62 |
| (7) Flavor | q.s. |
| (2) was dissolved in (1), the resultant solution was added to the mixture (3), (4) to be emulsified, and the emulsion then mixed with (5), (6), (7). | |

Example 17: Hair Mousse (Trademark)

| | |
|---|---|
| (1) Octamethylcyclotetrasiloxane | 13% |
| (2) Dimethylpolysiloxane ($R_1$ and $R_2$ are methyl groups, n = 10000) | 2 |
| (3) Glycerol | 1 |
| (4) Polyethylene (120) hardened castor oil ester | 2 |
| (5) Ethanol | 10 |
| (6) Deionized water | 62 |
| (7) N-butane | 7 |
| (8) Flavor | q.s. |
| (2) was dissolved in (1), the resultant solution was added to a mixture of (3) and (4) to be emulsified, and the emulsion then mixed with (5), (6), and (7). This mixture was charged into an aerosol container, and after attaching a valve, (8) was filled therein. | |

Example 18: Hair Cream

| | |
|---|---|
| (1) Decamethylcyclohexasiloxane | 28% |
| (2) Dimethylpolysiloxane ($R_1$ and $R_2$ in the formula (I) are methyl groups, n = 10000) | 6 |
| (3) Glycerol | 3 |
| (4) Polyethylene (120) hardened castor oil ester | 3 |
| (5) Ethanol | 10 |
| (6) Deionized water | 57 |
| (7) Polyvinyl alcohol | 1 |
| (8) Flavor | q.s. |
| (2) was dissolved in (1), the resultant solution was added to a mixture of (3) and (4) to be emulsified, and the emulsion was mixed with (5), (6), (7), and (8). | |

Example 19: Hair Oil

| | |
|---|---|
| (1) Decamethylcyclopentasiloxane | 60.0% |
| (2) Dimethylpolysiloxane (5 cps) | 20.0 |
| (3) Dimethylpolysiloxane ($R_1$ and $R_2$ in the formula (I) are methyl groups, n = 7000) | 10.0 |
| (4) Ethanol | 10 |
| (5) Flavor | q.s. |
| (1) - (5) were dissolved and mixed while stirring at 70 to 80 °C. A viscous liquid hair oil with a viscosity of 500 cps and a good transparency was obtained. | |

Comparative Example 1: Hair Oil

| | |
|---|---|
| (1) Decamethylcyclopentasiloxane | 60.0% |
| (2) Dimethylpolysiloxane (5 cps) | 30.0 |
| (3) Ethanol | 10 |
| (4) Flavor | q.s. |
| (1) - (4) were dissolved and mixed while stirring at 70 to 80 °C. A viscous liquid hair oil with a viscosity of 500 cps and a good transparency was obtained. | |

Comparative Example 2: Liquid Hair Conditioner

| | |
|---|---|
| (1) Vinylpyrrolidone/vinyl acetate copolymer | 2.0% |
| (2) Propylene glycol | 2.0 |
| (3) Ethanol | 40.0 |
| (4) Deionized water | 56.0 |

After (1) was dissolved in (3), the thus-obtained solution was added thereto, together with (2) to (4), to obtain a transparent liquid hair conditioner.

Evaluation Test

The properties and effects of the hair cosmetic compositions to be used in accordance with the present invention and obtained in Examples 16 - 19 and the conventional products obtained in Comparative

Examples 1 - 2 as described above are shown below.

1. The split hair generation preventive effect was evaluated. Bundles of hair collected from the same person were prepared, the samples were respectively applied to each bundle, and the number of split hairs generated by a mechanical brushing of 10000 strokes was counted.

| Sample | Number of split hairs generated |
|---|---|
| Example 17 | 100/1300 |
| Example 19 | 30/1300 |
| Comparative Example 1 | 200/1300 |
| Uncoated | 300/1300 |

The products to be used in accordance with the present invention exhibited a remarkable split hair generation prevention effect.

2. The effect of split hair generation prevention in actual use was evaluated. A panel consisting of six members was requested to use the hair oil of Example 19 on one half of the hair on the head and a sample of Comparative Example 1 on the other half, every day, and the amounts of split hairs generated on the corresponding sides after two months were compared.

| Panel No. | Example 19 | Comparative Example 1 |
|---|---|---|
| A | ± | - |
| B | + | - |
| C | + | ± |
| D | ++ | ± |
| E | ++ | ± |
| F | ++ | ± |

++ ... Very few

\+ ... Few

± ... Some

\- ... Many

It was found that the generation of split hair was reduced in the hair on which the compositions to be used in accordance with the present invention had been applied.

3. The split hair repairing effect was determined. The composition of Example 16 was used on the hair of panel members having many split hairs, and the effect of preventing splitting of the hairs was observed.

| Panel No. | Conspicuous hair splitting |
|---|---|
| G | + |
| H | + |
| I | ++ |
| J | ++ |

++ Not conspicuous

It was found that the use of the compositions to be used in accordance with the present invention substantially prevented hair splitting.

4. The smoothness was evaluated by a friction measuring machine, and the reduction of the frictional coefficient of the hair was determined.

| Sample | Frictional coefficient |
|---|---|
| Example 17 | 0.25 |
| Example 19 | 0.2 |
| Comparative Example 1 | 0.35 |
| Comparative Example 2 | 0.6 |
| Uncoated | 0.5 |

The smaller frictional coefficient represented a greater smoothness, and as can be seen, hair became smoother when the compositions to be used in accordance with the present invention had been used.

## Claims

**1.** The use for preventing the generation of split hair or torn hair and for repairing damaged hair of a hair cosmetic composition comprising at least one high molecular weight silicone having the formula (I):

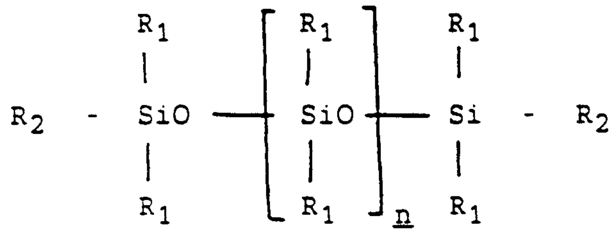

(I)

where each $R_1$ represents methyl or one or more of the $R_1$s represents phenyl and the remainder represent methyl, each $R_2$ represents methyl or hydroxy, and n represents an integer of 3000 to 20000, said composition being free of any dimethicone copolyol.

**2.** The use as claimed in claim 1, wherein the hair cosmetic composition contains a low boiling-point oil in an amount necessary for dissolving the high molecular weight silicone, said low boiling-point oil comprising a low boiling-point cyclic silicone having the formula:

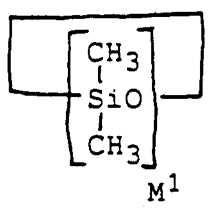

where $M^1$ is an integer of 3 to 7.

**3.** The use as claimed in claim 2, wherein the cyclic silicone is octamethylcyclotetrasiloxane, decamethylcyclopentasiloxane or dodecamethylcyclohexasiloxane.

**4.** The use as claimed in claim 1, wherein the hair cosmetic composition contains a low boiling-point oil in an amount necessary for dissolving the high molecular weight silicone, said low boiling-point oil comprising a low boiling-point linear silicone having the formula:

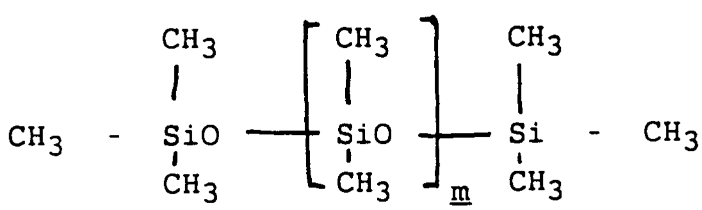

where m is zero or an integer of 1 to 5.

**5.** The use as claimed in claim 1, wherein the hair cosmetic composition contains a low boiling-point oil in an amount necessary for dissolving the high molecular weight silicone, said low boiling-point oil comprising an isoparaffinic hydrocarbon having a boiling-point at standard pressure of 60 to 260 °C.

**6.** The use as claimed in any of claims 1 to 5, wherein the hair cosmetic composition is an emulsified cosmetic composition.

**7.** The use as claimed in any of claims 1 to 6, wherein the emulsified cosmetic composition contains a nonionic surfactant.

**8.** The use as claimed in claim 6, wherein the emulsified cosmetic composition contains a cationic surfactant.

**9.** The use as claimed in claim 6, wherein the emulsified cosmetic composition contains an anionic surfactant.

**10.** The use as claimed in any of claims 6 to 9, wherein the emulsified cosmetic composition in an emulsified cosmetic composition which is emulsified by mixing a mixture of a water-soluble polyhydric alcohol and a surfactant with an oil phase.

**11.** The use of a non-emulsified or non-aqueous hair cosmetic composition for preventing generation of split hair or torn hair and for repairing damaged hair, said composition comprising at least one high molecular weight silicone having the formula (I):

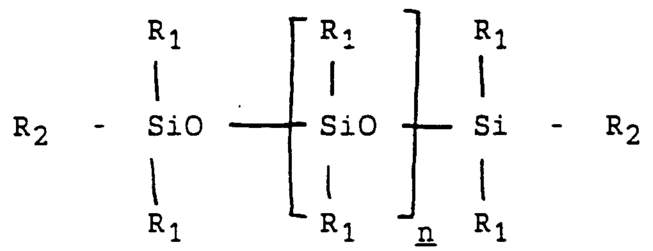 (I)

wherein each $R_1$ represents methyl or one or more of the $R_1$s represents phenyl and the remainder represent methyl, each $R_2$ represents methyl or hydroxy, and $\underline{n}$ represents an integer of 3000 to 20000, said composition being free from any dimethicone copolyol.

**12.** The use as claimed in any of claims 1 to 11, wherein the hair cosmetic composition comprises:
(A) 0.1% to 25% by weight of at least one high molecular weight silicone having the formula (I) defined in claim 1;
(B) 1% to 98.9% by weight of at least one cyclic silicone; and
(C) 1% to 98.9% by weight of trichlorotrifluoroethane and/or tetrachlorodifluoroethane.

**13.** The use as claimed in any of claims 1 to 11, wherein the hair cosmetic composition comprises:
(A) 0.1% to 25% by weight of at least one high molecular weight silicone having the formula (I) defined in claim 1;
(B) 45% to 98.9% by weight of at least one cyclic silicone; and
(C) 1% of 30% by weight of at least one lower alcohol.

**14.** The use as claimed in any of claims 1 to 11, wherein the hair cosmetic composition is in the form of an aerosol composition and comprises:
(A) 0.1% to 25% by weight of at least one high molecular weight silicone having the formula (I) defined in claim 1;
(B) 1% to 98.9% by weight of trichlorotrifluoroethane and/or tetrachlorodifluoroethane; and
(C) 5% of 95% by weight of an aerosol propellant.

**15.** The use as claimed in claim 14, wherein the aerosol propellant is at least one compound selected from trichlorofluoromethane, dichlorodifluoromethane, dichlorotetrafluoroethane, monochlorodifluoromethane, dimethylether, propane, isobutane and n-butane.

**16.** The use of a silicone for the production of a hair cosmetic composition for preventing the generation of split hair or torn hair and for repairing damaged hair, said composition comprising at least one high molecular weight silicone having the formula (I):

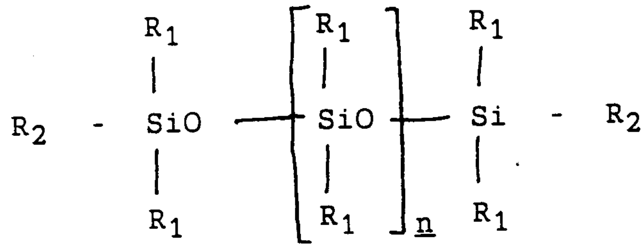

(I)

where each $R_1$ represents methyl or one or more of the $R_1$s represents phenyl and the remainder represent methyl, each $R_2$ represents methyl or hydroxy, and n represents an integer of 3000 to 20000, said composition being free of any dimethicone copolyol.

**17.** The use as claimed in claim 16, wherein the hair cosmetic composition is a composition as defined in any of claims 2 to 15.

**Patentansprüche**

**1.** Verwendung eines haarkosmetischen Mittels zur Vorbeugung der Bildung von gesplissenem Haar und gebrochenem Haar und zur Behandlung von beschädigtem Haar, enthaltend wenigstens ein Silikon mit hohem Molekulargewicht der Formel (1)

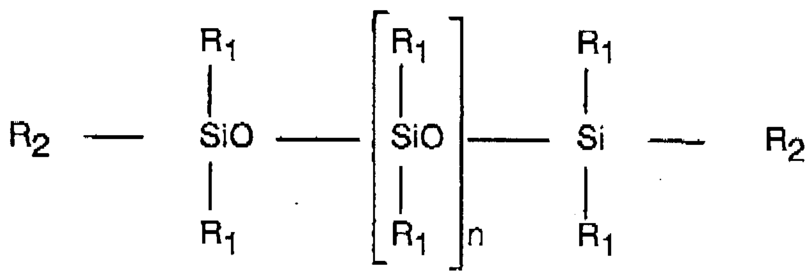

(I)

worin jedes $R_1$ Methyl bedeutet oder eines oder mehrere von $R_1$ Phenyl und der Rest Methyl bedeuten, jedes $R_2$ Methyl oder Hydroxy und n eine ganze Zahl von 3000 bis 20000 bedeuten, wobei diese Zusammensetzung frei von einem Dimethiconcopolyol ist.

**2.** Verwendung nach Anspruch 1, worin das haarkosmetische Mittel ein niedrigsiedendes Öl in einer Menge enthält, die erforderlich ist, das hochmolekulare Silikon zu lösen, wobei das niedrigsiedende Öl ein cyclisches niedrigsiedendes Silikon der Formel

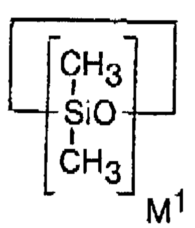

enthält, worin $M^1$ eine ganze Zahl von 3 bis 7 ist.

**3.** Verwendung nach Anspruch 2, worin das cyclische Silikon Octamethylcyclotetrasiloxan, Decamethylcyclopentasiloxan oder Dodecamethylcyclohexasiloxan ist.

**4.** Verwendung nach Anspruch 1, worin das haarkosmetische Mittel ein niedrigsiedendes Öl in einer Menge enthält, die erforderlich ist, das hochmolekulare Silikon zu lösen, wobei das niedrigsiedende Öl

ein geradkettiges, niedrigsiedendes Silikon der Formel

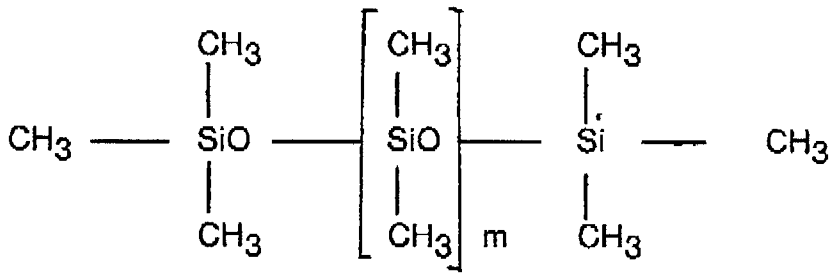

enthält, worin m 0 oder eine ganze Zahl von 1 bis 5 ist.

**5.** Verwendung nach Anspruch 1, worin das haarkosmetische Mittel ein niedrigsiedendes Öl in einer Menge enthält, die erforderlich ist, das hochmolekulare Silikon zu lösen, wobei das niedrigsiedende Öl einen Isoparaffinkohlenwasserstoff mit einem Siedepunkt bei Normaldruck von 60 bis 260 °C enthält.

**6.** Verwendung nach einem der Ansprüche 1 bis 5, worin das haarkosmetische Mittel ein emulgiertes kosmetisches Mittel ist.

**7.** Verwendung nach einem der Ansprüche 1 bis 6, worin das emulgierte kosmetische Mittel ein nichtionisches Tensid enthält.

**8.** Verwendung nach Anspruch 6, worin das emulgierte kosmetische Mittel ein kationisches Tensid enthält.

**9.** Verwendung nach Anspruch 6, worin das emulgierte kosmetische Mittel ein anionisches Tensid enthält.

**10.** Verwendung nach einem der Ansprüche 6 bis 9, worin das emulgierte kosmetische Mittel emulgiert wurde durch Vermischen einer Mischung eines wasserlöslichen Polyhydroxyalkohols und eines Tensids mit einer Ölphase.

**11.** Verwendung eines nicht emulgierten oder nicht nicht wäßrigen haarkosmetischen Mittels zur Vorbeugung der Bildung von gesplissenem Haar und gebrochenem Haar und zur Behandlung von beschädigtem Haar, wobei diese Zusammensetzung wenigstens ein hochmolekulares Silikon der Formel (I)

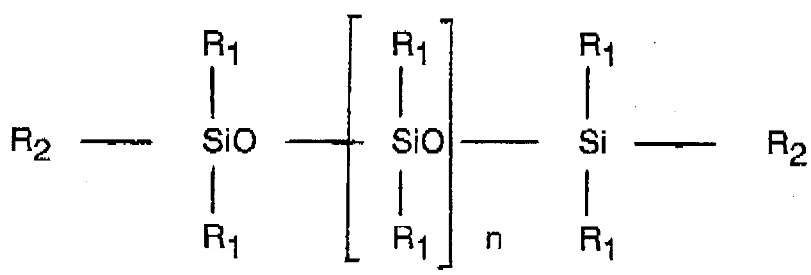 (I)

enthält, worin R1 Methyl bedeutet oder eines oder mehrere von $R_1$ Phenyl und der Rest Methyl bedeuten, jedes $R_2$ Methyl oder Hydroxy und n eine ganze Zahl von 3000 bis 20000 bedeuten, wobei diese Zusammensetzung frei von einem Dimethiconcopolyol ist.

**12.** Verwendung nach einem der Ansprüche 1 bis 11, worin das haarkosmetische Mittel
(A) 0,1 bis 25 Gew. % wenigstens eines hochmolekularen Silikons der Formel (I), wie in Anspruch 1 definiert,
(B) 1 bis 98,9 Gew. % wenigstens eines cyclischen Silikons und
(C) 1 bis 98,9 Gew. % Trichlortriflourethan und/oder Tetrachlordiflourethan enthält.

**13.** Verwendung nach einem der Ansprüche 1 bis 11, worin das haar-kosmetische Mittel

(A) 0,1 bis 25 Gew. % wenigstens eines hochmolekularen Silikons der Formel (I), wie in Anspruch 1 definiert,
(B) 45 bis 98,9 Gew. % wenigstens eines cyclischen Silikons und
(C) 1 bis 30 Gew. % wenigstens eines niederen Alkohols enthält.

**14.** Verwendung nach einem der Ansprüche 1 bis 11, worin das haarkosmetische Mittel in Form eines Aerosols vorliegt und
(A) 0,1 bis 25 Gew. % wenigestens eines hochmolekularen Silikons der Formel (I) wie in Anspruch 1 definiert.
(B) 1 bis 98,9 Gew. % Trichlortrifluorethan und/oder Tetrachlordifluorethan und
(C) 5 bis 95 Gew. % eines Aerosol-Treibgases. enthält.

**15.** Verwendung nach Anspruch 14, worin das Aerosol-Treibgas wenigstens eine Verbindung, ausgewählt aus Trichlorfluormethan, Dichlordifluormethan, Dichlortetrafluorethan, Monochlordifluormethan, Dimethylether, Propan, Isobu n-Butan ist.

**16.** Verwendung eines Silikons zur Herstellung eines haarkosmetischen Mittels zur Vorbeugung der Bildung von gesplissenem Haar und gebrochenem Haar und zur Behandlung von beschädigtem Haar, wobei diese Zusammensetzung wenigstens ein hochmolekulares Silikon der Formel (I)

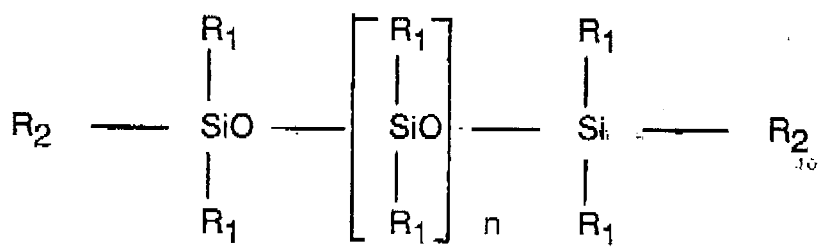 (I)

enthält, worin $R_1$ Methyl bedeutet oder eines oder mehrere von $R_1$ Phenyl und der Rest Methyl bedeuten, jedes $R_2$ Methyl oder Hydroxy und $\underline{n}$ eine ganze Zahl von 3000 bis 20000 bedeuten, wobei die Zusammensetzung frei von einem Dimethiconcopolyol ist.

**17.** Verwendung nach Anspruch 16, worin das haarkosmetische Mittel eine Zusammensetzung ist, wie sie in einem der Ansprüche 2 bis 15 bezeichnet ist.

**Revendications**

**1.** L'utilisation pour la prévention de l'apparition de cheveux fourchus ou cheveux abîmés et pour le traitement des cheveux endommagés, d'une composition cosmétique capillaire comprenant au moins un silicone de poids moléculaire élevé représenté par la formule (I)

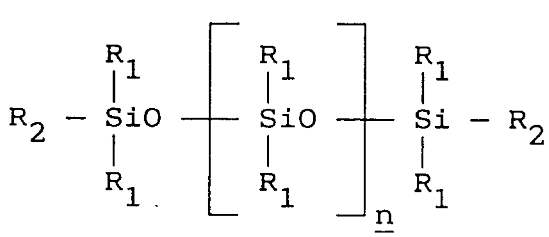 (I)

dans laquelle:
chaque groupe $R_1$ représente un radical méthyle ou un ou plusieurs groupes $R_1$ représentent un radical phényle et le reste des groupes $R_1$ représente un radical méthyle;
chaque groupe $R_2$ représente un radical méthyle ou hydroxy, et
n représente un nombre entier compris entre 3000 et 20 000;

ladite composition étant exempte de tout copolyol de diméthicone.

**2.** Utilisation selon la revendication 1, caractérisée en ce que la composition cosmétique capillaire contient une huile de faible point d'ébullition en une quantité nécessaire pour dissoudre le silicone de haut poids moléculaire et ladite huile de faible point d'ébullition comprenant un silicone cyclique de faible point d'ébullition de formule:

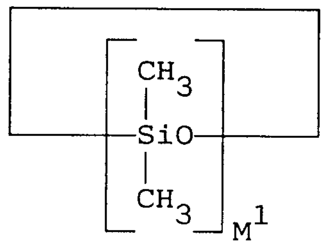

dans laquelle:

$M^1$ représente un nombre entier compris entre 3 et 7.

**3.** Utilisation selon la revendication 2, caractérisée en ce que le silicone cyclique est l'octaméthylcyclotétrasiloxane, le décaméthylcyclopentasiloxane ou le dodécaméthylcyclohexasiloxane.

**4.** Utilisation selon la revendication 1, caractérisée en ce que la composition cosmétique capillaire contient une huile de faible point d'ébullition en une quantité nécessaire pour dissoudre le silicone de haut poids moléculaire, ladite huile de faible point d'ébullition comprenant un silicone linéaire de faible point d'ébullition de formule:

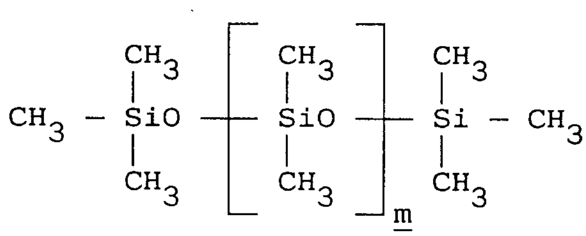

(I)

dans laquelle:

m représente 0 ou un nombre entier compris 1 et 5.

**5.** Utilisation selon la revendication 1, caractérisée en ce que la composition cosmétique capillaire contient une huile de faible point d'ébullition en une quantité nécessaire pour dissoudre le silicone de haut poids moléculaire, ladite huile de faible point d'ébullition comprenant un hydrocarbure isoparaffinique ayant un point d'ébullition à la pression standard compris entre 60 ° C et 260 ° C.

**6.** Utilisation selon l'une quelconque des revendications 1 à 5, caractérisée en ce que la composition cosmétique capillaire est une composition cosmétique émulsifiée.

**7.** Utilisation selon l'une quelconque des revendications 1 à 6, caractérisée en ce que la composition cosmétique émulsifiée contient un tensioactif non-ionique.

**8.** Utilisation selon la revendication 6, caractérisée en ce que la composition cosmétique émulsifiée contient un tensioactif cationique.

**9.** Utilisation selon la revendication 6, caractérisée en ce que la composition cosmétique émulsifiée contient un tensioactif anionique.

**10.** Utilisation selon l'une quelconque des revendications 6 à 9, caractérisée en ce que la composition cosmétique émulsifiée est émulsifiée par mélange d'un mélange d'alcool polyvalent hydrosoluble et d'un tensioactif avec une phase huileuse.

**11.** Utilisation d'une composition cosmétique capillaire non-aqueuse ou non-émulsifiée pour la prévention de l'apparition de cheveux fourchus ou de cheveux abîmés et pour le traitement des cheveux endommagés, ladite composition comprenant au moins un silicone de haut poids moléculaire représenté par la formule (I):

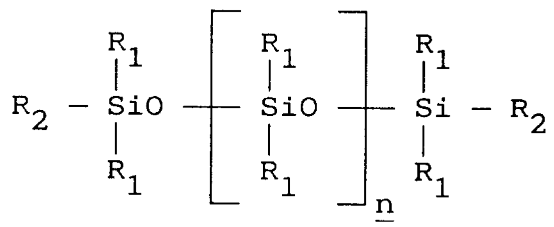

(I)

dans laquelle:

chaque groupe $R_1$ représente un radical méthyle ou un ou plusieurs groupes $R_1$ représentent un radical phényle et le reste des groupes $R_1$ représente un radical méthyle;

chaque groupe $R_2$ représente un radical méthyle ou hydroxy, et

n représente un nombre entier compris entre 3000 et 20 000;

ladite composition étant exempte de tout copolyol de diméthicone.

**12.** Utilisation selon l'une quelconque des revendications 1 à 11, caractérisée en ce que la composition cosmétique capillaire comprend:

(A) de 0,1 à 25% en poids d'au moins un silicone de haut poids moléculaire représenté par la formule (I) définie dans la revendication 1;
(B) de 1 à 98,9% en poids d'au moins un silicone cyclique; et
(C) de 1 à 98,9% en poids de trichlorotrifluoroéthane et/ou tétrachlorodifluoroéthane.

**13.** Utilisation selon l'une quelconque des revendications 1 à 11, caractérisée en ce que la composition cosmétique capillaire comprend:

(A) de 0,1 à 25% en poids d'au moins un silicone de haut poids moléculaire représenté par la formule (I) définie dans la revendication 1;
(B) de 45 à 98,9% en poids d'au moins un silicone cyclique; et
(C) de 1 à 30% en poids d'au moins un alcool inférieur.

**14.** Utilisation selon l'une quelconque des revendications 1 à 11, caractérisée en ce que la composition cosmétique capillaire est sous la forme d'une composition aérosol qui comprend:

(A) de 0,1 à 25% en poids d'au moins un silicone de haut poids moléculaire représenté par la formule (I) définie dans la revendication 1;
(B) de 1 à 98,9% en poids de trichlorotrifluoroéthane et/ou tétrachlorodifluoroéthane; et
(C) de 5 à 95% en poids d'un gaz propulseur pour aérosol.

**15.** Utilisation selon la revendication 14, caractérisée en ce que le gaz propulsé par aérosol correspond à au moins l'un des composés sélectionné dans le groupe constitué par: trichlorofluorométhane, dichlorodifluorométhane, dichlorotétrafluoroéthane, monochlorodifluorométhane, diméthyléther, propane, isobutane et n-butane.

**16.** Utilisation d'un silicone pour la préparation d'une composition cosmétique capillaire permettant la prévention de l'apparition de cheveux fourchus ou cheveux abîmés et le traitement des cheveux endommagés, ladite composition comprenant au moins un silicone de haut poids moléculaire de formule (I):

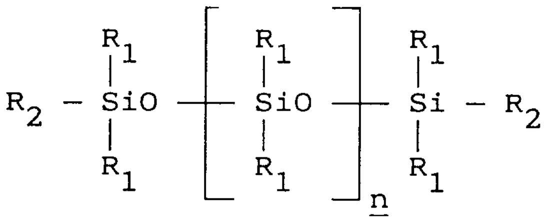

(I)

dans laquelle:

chaque groupe $R_1$ représente un radical méthyle ou un ou plusieurs groupes $R_1$ représentent un radical phényle et le reste des groupes $R_1$ représente un radical méthyle;

chaque $R_2$ représente un radical méthyle ou hydroxy, et

n représente un nombre entier compris entre 3000 et 20 000;

ladite composition étant exempte de tout copolyol de diméthicone.

**17.** Utilisation selon la revendication 16, caractérisée en ce que la composition cosmétique capillaire correspond à une composition telle que définie dans l'une quelconque des revendications 2 à 15.